**Europäisches Patentamt**

**European Patent Office**

**Office européen des brevets**

(19)

(11) Publication number: **0 202 420 B1**

(12) EUROPEAN PATENT SPECIFICATION

(45) Date of publication of patent specification: **02.01.91**

(51) Int. Cl.⁵: **C 07 C 321/14,** C 07 C 321/04, C 07 C 319/14

(21) Application number: **86103720.8**

(22) Date of filing: **19.03.86**

(54) Process for the manufacture of dialkyl disulfides.

(30) Priority: **13.05.85 US 733551**

(43) Date of publication of application:
**26.11.86 Bulletin 86/48**

(45) Publication of the grant of the patent:
**02.01.91 Bulletin 91/01**

(84) Designated Contracting States:
**AT BE CH DE FR GB IT LI LU NL SE**

(56) References cited:
**EP-A- 171 092**
**US-A-2 910 506**
**US-A-3 340 324**

(73) Proprietor: **ATOCHEM NORTH AMERICA, INC. (a Pennsylvania corp.)**
**Three Parkway**
**Philadelphia Pennsylvania 19102 (US)**

(72) Inventor: **Buchholz, Bernard**
**634 School Rd**
**Blue Bell, PA 19422 (US)**
Inventor: **Dzierza, Edward John**
**661 Kismet Rd**
**Philadelphia, PA 19115 (US)**
Inventor: **Baltrus, John Robert**
**902 Greenbrier Drive**
**Norristown, PA 19401 (US)**

(74) Representative: **Kraus, Walter, Dr. et al**
**Patentanwälte Kraus, Weisert & Partner**
**Thomas-Wimmer-Ring 15**
**D-8000 München 22 (DE)**

Courier Press, Leamington Spa, England.

**Description**

Background of the invention

This invention relates to a continuous process for the manufacture of dialkyl disulfides by reacting an alkyl alcohol with hydrogen sulfide over a solid, particulate catalyst, in one reactor zone and then passing the reactor effluent into a second reaction zone, where it is reacted as a vapor with elemental molten sulfur in the presence of the same or different solid, particulate catalyst. More particularly, it relates to a two-reactor process for the continuous manufacture of a dialkyl disulfide from a $C_1$—$C_{12}$ alkyl alcohol, hydrogen sulfide, and sulfur, in the presence of solid particulate catalysts.

The process reactions are represented by the following equations:

$$\text{catalyst (1) } 2\ ROH + 2\ H_2S \rightarrow 2\ RSH + 2\ H_2O \text{ (reactor 1)}$$

$$\text{catalyst (2) } 2\ RSH + S \rightarrow RSSR + H_2S \text{ (reactor 2)}$$

$$\text{(3) } 2\ ROH + H_2S + S \rightarrow RSSR + 2\ H_2O \text{ (overall process)}$$

When R is methyl, for example, the process can be utilized to prepare dimethyl disulfide (DMDS) from methanol, hydrogen sulfide, and elemental sulfur, according to equation (3) above. DMDS is a well known article of commerce, being used as a sulfiding agent for the pre-treatment and post-regenerative treatment of hydrodesulfurization catalysts in petroleum refining, as a down-hole sulfur solvent for oil wells, and as a chemical intermediate in the manufacture of agricultural compounds in lieu of methyl mercaptan.

Prior art

It is known that disulfides are produced by the oxidation of mercaptans with sulfur, according to equation (4), especially in the presence

$$\text{(4)} \qquad\qquad 2\ RSH + S \rightarrow RSSR + H_2S$$
$$(R = \text{alkyl or aryl})$$

of alkali, ammonia, or an amine (E. E. Reid, *Organic Chemistry of Bivalent Sulfur*, Volume 1, p. 121, Chemical Publishing Co., Inc., New York (1958). The liquid-phase reaction of mercaptans with sulfur as the oxidizing agent using alkali or amine catalysts is reported in U.S. Patent 3,314,999 & U.S. Patent 3,755,461. In the above method, it is first necessary to manufacture and isolate the mercaptan before oxidizing it with sulfur to the corresponding disulfide.

Processes for the manufacture of mercaptans from alcohols and hydrogen sulfide, according to equation (5), where R is alkyl

$$\text{(5)} \qquad\qquad ROH + H_2S \rightarrow RSH + H_2O$$

or aryl, are known and shown, for example, in U.S. Patent 3,035,097.

EP—A—0 171 092 discloses a process for the continuous preparation of dialkyl disulfides in a single reactor by reacting an alkyl alcohol, hydrogen sulfide and sulfur at an elevated temperature and in the presence of a solid particulate catalyst.

This invention relates to a process for preparing di($C_1$—$C_{12}$)alkyl disulfides comprising continuously reacting a $C_1$—$C_{12}$ alkyl alcohol and hydrogen sulfide in a molar ratio of 1:2 to 1:20 in contact with a solid, particulate catalyst in a first reaction zone at a temperature ranging from 100 to 500°C, at a pressure ranging between atmospheric and 35 bar (500 psig) and a molar velocity of between 50 and 500 gram-moles of alcohol per kilogram of catalyst per 24 hours, continuously passing the crude mercaptan product of the reaction and molten sulfur at a molar ratio of from 1:0.05 to 1:2 in contact with a solid, particulate catalyst in a second reaction zone at a temperature ranging from 125 to 400°C, and pressure ranging from atmospheric to 42.5 bar (600 psig) and recovering the di ($C_1$—$C_{12}$) alkyl disulfide, wherein the catalyst in the second reaction zone is a Type X, Y or L zeolite.

A method of manufacturing dialkyl disulfides in a continuous two-reactor process, using solid particulate catalysts in each reactor, has been found which process possesses an improved selectivity for the formation of dialkyl disulfides from alkyl alcohols, hydrogen sulfide, and sulfur.

One advantage of this process over prior art processes is that it produces dialkyl disulfides from alkyl alcohols, rather than alkyl mercaptans, as a raw material, resulting in a substantial cost savings. Another advantage of this process is that the dialkyl disulfides can be manufactured with very little formation of unwanted byproduct dialkyl sulfides or carbon disulfide. Still another advantage is that the dialkyl polysulfides, which are formed as byproducts in this process, can be totally recycled to the second reactor where they react with the intermediate alkyl mercaptan to form additional dialkyl disulfide. Still another advantage is that high dialkyl disulfide production rates can be sustained for long periods of time without the necessity for periodic air-regeneration of the catalyst to remove coke and tars.

The $C_1$—$C_{12}$ alkyl alcohols used in the process of this invention include, for example, methanol,

2

ethanol, propanol, butanol, pentanol, hexanol, heptanol, octanol, decanol, dodecanol and isomeric forms of these normal alcohols. Preferably, the $C_1$—$C_6$ alkyl alcohols are used in the process, more preferably the $C_1$—$C_4$ alkanols and most preferably, methanol.

Any of a variety of solid, particulate catalysts, preferably aluminum-containing catalysts, are used in both of the reaction zones of the process of this invention. Alumina, silica, thoria, or alumina promoted with an alkali metal tungstate or alumina promoted with an alkali metal heteropoly acid salt, such as potassium phosphotungstate, can be used to convert the $C_1$ to $C_{12}$ alkyl alcohol to $C_1$ to $C_{12}$ alkyl mercaptan. The catalysts described at column 2, line 43 through a column 4, line 24 of U.S. Patent No. 3,035,097 are some of the catalysts suitable for this invention and this portion of the '097 patent is incorporated herein by reference.

The aluminum-containing catalysts of the second reaction-zone, are preferably synthetic aluminosilicates characterized by high uniformity, well-defined pore size, large surface area, and complete crystallinity. The structures of the zeolite catalysts are described in Union Carbide's booklet F-08 entitled *"Zeolite Molecular Sieve Catalysts"* and D. W. Breck, *Zeolite Molecular Sieves*, (1974), John Wiley & Sons, New York. Various types of zeolite catalysts are manufactured, for example, by Akzo Chemie, Air Products (Houdry), Norton, PQ Corporation, United Catalysts, and Union Carbide.

The basic unit of the synthetic zeolites is composed of silicon and aluminum atoms tetrahedrally coordinated with four oxygen atoms. Since the aluminum atoms are trivalent, they have a net negative charge when bonded with 4 oxygen atoms (A104-). This charge is balanced by a cation, such as Na+, K+, or H+ in the as-synthesized zeolites. These cations can be exchanged with other metals or cations. For example, a divalent cation such as cobalt will replace 2 univalent cations, while a trivalent cation such as chromium, lanthanum, or cerium will replace 3 univalent cations. It is thus possible to replace the alkali metal cations, Na+ or K+, with catalytically more active cations such as Ag+1, Co+2, Ni+2, Mo+2 (or +3), Fe+2 (or +3), Cr+3, La+3, etc., if desired. However, the alkali metal cations are preferred for this invention, with the catalyst typically containing about 13 percent by weight of the alkali metal, expressed as the alkali metal oxide (e.g., $Na_2O$, $K_2O$).

Although many factors influence the catalytic activity of these zeolites, the three most important are, 1) the open framework structure with its attendant pore size, 2) the $SiO_2$:$Al_2O_3$ ratio of the framework, and 3) the cations. As in most catalytic processes, the large-pore zeolites having pore openings in the range of 7 to 10 Angstroms are most useful. The most preferred are Type X, Type Y, and Type L zeolites. Type X has a chemical composition expressed in terms of oxide ratios of $Na_2O$:$Al_2O_3$:2—$3SiO_2$ with a typical unit cell composition in the hydrated state of

$$Na_{86}[AlO_2)_{86}(SiO_2)_{106}] \cdot 264\ H_2O.$$

Type Y, on the other hand, has a composition of $Na_2O$:$Al_2O_3$:>3—6 $SiO_2$. When the $SiO_2$:$Al_2O_3$ molar ratio is 4.8, the hydrated unit cell composition is

$$Na_{56}[(AlO_2)_{56}(SiO_2)_{136}] \cdot 264\ H_2O.$$

Type L, more siliceous than Type X and Type Y, also has a pore size in the 0.7—1.0 mm (7 to 10 Angstroms) range.

An important building block of these zeolites is the sodalite cage, a truncated octahedron unit consisting of 24 ($SiO_4$, $AlO_4$) units. In Type X and Type Y zeolites, the sodalite cages are connected through 4 of the 8 hexagonal faces in a tetrahedral arrangement. The pores thus created are defined by a 12-member ring of oxygen atoms, approximately 0.7—0.9 mm (7 to 9 Angstroms) in size, opening into a central cavity of 1.1 mm (11 Angstroms) in diameter.

The more preferred synthetic zeolites are Types X and Y because of their larger pore sizes. The ability of the Type Y to withstand higher temperatures without losing its crystalline structure makes it the most preferred zeolite catalyst for the second reaction zone of this invention.

The zeolites, as prepared, generally contain as the cation 13 percent by weight sodium (as $Na_2O$) or equivalent amount of other alkali metal (as $Me_2O$). As explained above, this cation may be replaced with other cations to reduce the sodium content. In this invention, however, the most preferred catalyst contains sodium as the cation, with a sodium content of at least 3 percent, preferably more than 5 percent, more preferably greater than 10 percent, and most preferably at the 13 percent by weight (as $Na_2O$) level.

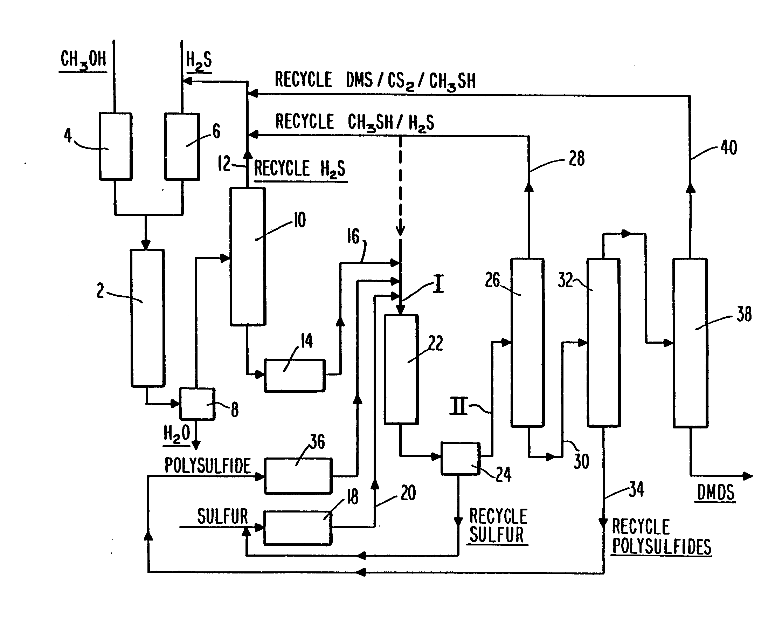

## The drawing

An example of the process of this invention is depicted in the drawing which is a flow diagram for the manufacture of dialkyl disulfide represented by dimethyl disulfide (DMDS). Methanol and hydrogen sulfide are fed continuously in a molar ratio ranging from 1:2 to 1:20, preferably from 1:6 to 1:10 to a first reactor 2, the excess hydrogen sulfide being used to depress the formation of byproduct dimethyl sulfide. The reactants are heated and vaporized in preheaters 4 and 6, mixed, and charged to reactor 2 wherein the reaction takes place in the presence of a solid, particulate catalyst, e.g., alumina with or without potassium phosphotungstate promoter, at a pressure ranging between atmospheric and 35 bar (500 psig), preferably between 6.9 and 27.5 bar (100 and 400 psig), and at a temperature ranging from 100 to 500°C, preferably from 250 to 400°C. The reaction temperature is determined by the catalyst bed temperature. The molar velocity of the alcohol may vary over a wide range but will usually be between 50 and 500, preferably between 100 and 150, gram-moles of alcohol vapor per kilogram of catalyst per 24 hours (at STP). The volume of the catalyst in the first reaction zone is adjusted to produce mercaptan at the desired rate for passage of the crude mercaptan to the second reaction zone.

Most of the byproduct water is removed in the liquid-phase water-separator 8. A major portion of the hydrogen sulfide in the effluent from reactor 2 is separated from the effluent mixture in high peresusre separator 10 and returned via line 12 to reactor 2. Crude mercaptan in the effluent stream passing from the bottom of separator 10 is preheated at 14 and forwarded through line 16 to the second reactor. Fresh sulfur is also introduced to the second reactor through preheater 18 via line 20. Molten sulfur is fed to second reactor 22 in a molar ratio that is preferably 0.15 for each mole of mercaptan which provides less sulfur than is required to meet the stoichiometric requirement for the equation:

$$2\ CH_3SH + S \rightarrow CH_3SSCH_3 + H_2S$$

This sulfur deficiency minimizes polysulfide formation. The preheated reactants are mixed, at point I, and charged to the reactor 22 wherein they are subjected to elevated temperatures in the range of 125 to 250°C and pressures from atmospheric to 42.5 bar (600 psig) in the presence of a particulate catalyst, preferably a Type X or Y zeolite, to effect reaction. Under the stated conditions, the crude methyl mercaptan is in the vapor phase and the elemental sulfur is in the liquid phase.

Any unreacted sulfur is separated in a knock-out pot 24 from the crude product issuing from the bottom of reactor 22. After sulfur separation, the crude product is passed into a series of distillation columns (or towers). Tpe first column 26 removes the low-boilers (largely unreacted methyl mercaptan and H₂S) through the overhead stream 28 and recycles them back to the reactor 2 or, alternatively, reactor 22. The bottom stream is then passed via line 30 to the second distillation tower 32 where the heavies, mostly polysulfides, are taken as bottoms and recycled through heater 36 back to second reactor 22 to react with the mercaptan to form more disulfide

$$(e.g.,\ CH_3SSSCH_3 + 2\ CH_3SH \rightarrow 2\ CH_3SSCH_3 + H_2S).$$

The remaining low-boilers, e.g., minor amounts of dimethyl sulfide and carbon disulfide, and the product, DMDS, are taken as an overhead and passed to the third tower 38. The high-purity product DMDS, is taken off from tower 38 as a bottoms material, while the low-boilers are taken overhead through line 40 for recycle back to the first reactor 2.

Operable conditions for the desired reaction to occur in the reactor 22 are the presence of a solid, particulate catalyst, a catalyst bed temperature in the range 125—400°C, and pressures ranging from atmospheric to 600 psig. The molar ratio of fresh sulfur and crude alkyl mercaptan from reactor 2 fed to the second reactor 22 may range from a 2 to 1 molar excess of sulfur over alkyl mercaptan to a 20 to 1 molar excess of alkyl mercaptan over sulfur. The molar ratios in the combined fresh-plus-recycle feed to the reactor 22 may, of course, be outside this range and will usually contain a substantial molar excess of alkyl mercaptan over sulfur, which may be as high as 20 to 1, to avoid excess polysulfide formation. The feed to the reactor 22 may also contain up to 50 percent by volume of an inert gas or mixture of inert cooling gases to provide sufficient heat removal from the catalyst zone. The inert gases may be nitrogen, methane, ethane, propane, butane, carbon dioxide, or any other gas that does not interfere with the reactions to produce the desired dialkyl disulfide. The rate at which the crude alkyl mercaptan is passed over the catalyst may range from about 100 to about 2000 gram-moles of alkyl mercaptan per kilogram of catalyst per 24 hours, or expressed in different units, from 100 to about 2000 pound-moles per 1000-pounds of catalyst per 24-hour day.

The preferred catalyst-bed temperatures (catalyst bed temperature equals reaction temperature) in reactor 22 are in the range 125—225°C, and the preferred pressures are in the range 50—375 psig. The preferred molar ratio of crude alkyl mercaptan to sulfur fed into reactor 22 is in the range 20/1 to 1/1, and is most preferably near the molar ratio of about 7/1. The preferred rate at which the crude alkyl mercaptan is passed over the catalyst is in the range 750—1250 gram-moles of alkyl mercaptan per kilogram of catalyst per 24 hours. The preferred catalyst is a Type Y zeolite having a sodium content of 13% by weight, expressed as Na₂O.

The following examples omit the first phase reaction of this process wherein methyl mercaptan is produced substantially in accordance with the process disclosed in U.S. 3,035,097 from methyl alcohol and hydrogen sulfide. Instead, a simulated crude product of the first phase reaction consisting of methyl mercaptan and hydrogen sulfide is fed to the second stage reactor along with sulfur (with and without recycle polysulfides) in the prescribed molar ratio. In the example, 14 runs are made to demonstrate the process of this invention using a Type Y zeolite catalyst containing 13 weight percent sodium expressed as Na₂O. The composition of the crude product, sampled at point II of the flow diagram of the Drawing, is determined by gas chromatographic (GC) analysis. The material balances across reactor 22, and the single-pass conversions of the crude methyl mercaptan to DMDS are calculated from the GC data.

**Example 1**

To simulate a fresh-plus-recycle feed mixture of the process, as indicated at point I of the flow diagram of the Drawing, when all byproduct dimethyl polysulfides are recycled, methyl mercaptan, $H_2S$, sulfur, and an approximately 80/20 by weight mixture of dimethyl trisulfide and dimethyl tetrasulfide, were pumped separately, as liquids, at appropriate rates to provide a continuous $CH_3SH/H_2S/S/DMS_x$ mixture in the desired molar ratios from 1/0.5/0.15/0.04 to 1/0.5/0.15/0.09.

The above reaction components were passed individually through stainless steel packed tubes installed in an electrically-heated preheater maintained at 200—225°C. The liquid polysulfides (80/20 mix) were blended with the vaporized gases in a static mixer before entering the reactor tube. The molten sulfur was then injected into the gas stream at the top of the reactor tube. The reactor is a 316 stainless steel tube, 2 inches in diameter (i.d.), and 36 inches in length, enclosed in an electrically heated vertical furnace. The catalyst is a Type Y zeolite containing 13% by weight sodium, expressed as $Na_2O$, in the form of a 1/8 inch extrudate sold by Union Carbide under a product designation LZ-Y52. It is arranged in a 6—9 inch fixed bed located centrally within the vertical reactor tube and maintained in the temperature range of 145—165°C. The exit stream was passed as a vapor into a stainless steel vessel maintained at 165°C to separate unreacted sulfur from the crude product stream. The effluent was then cooled by passing the crude product through a coil immersed in a cooling bath maintained at minus 5°C, such temperature being sufficient to completely liquify the crude reactor effluent. The liquified stream was then passed directly into a gas chromatograph for analysis. The stream was visually inspected to confirm complete liquification, passed through a back-pressure control-release valve and then into a closed-end vessel maintained at minus 50°C. The pressure in the reactor system was kept between 22.34 and 23.4 bar (325 and 340 psig), and the methyl mercaptan molar-velocity was maintained at 1000 gram-moles of $CH_3SH$ per kilogram of catalyst per 24 hours.

Two series of seven continuous runs (1—14), of approximately 12 hours duration each, were made using the Union Carbide LZ-Y52 sodium zeolite catalyst. The reaction conditions and production rates of the products are given in Table 1 for each run. A series of GC analyses of the effluent were made at point II of the Drawing during each run and averaged to obtain the production figures shown for each run. Other runs using the above described zeolite catalyst and varying process conditions are shown in Table 2 (Runs 15—23) and Table 3 (Runs 24—40).

Overall yields of DMDS, based on methanol, are calculated to be over 90% for this two-reactor process, with recycling, as illustrated in the Drawing, when operating at preferred conditions.

For the Type Y zeolite catalyst of high sodium content, Tables 1, 2, and 3 distinctly show the effect of operating conditions on the rate of product formation. In Table 1, the effect of the sulfur to methyl mercaptan ratio is clearly shown. On reducing the sulfur-to-mercaptan molar ratio to 0.2:1 or lower, no $CS_2$ by-product is produced. The conversion of methyl mercaptan to DMDS is calculated as the moles of DMDS produced times 2 divided by the moles of $CH_3SH$ fed. As shown in Table 1, 19 to 32 percent of the methyl mercaptan fed is converted in a single pass to DMDS, with no yield losses to dimethyl sulfide or $CS_2$. Although some of the conversions of methyl mercaptan to DMDS shown in Tables 2 and 3 are nearly equal to those shown in Table 1, the high production rate of byproduct $CS_2$, and the presence of dimethyl sulfide, shown in Tables 2 and 3, make the conditions employed less desirable.

**Example 2**

Example 1 is repeated, except that the catalyst used is Union Carbide's LZ-Y62 protonated zeolite (about 2.5% sodium expressed as $Na_2O$) and, except for the high $H_2S$ levels used, the operating conditions are comparable to those in Tables 2 and 3. The high $H_2S$ for each run was needed to reduce coke formation. In run 44, the conversion reached the level of single-pass conversion (22%) observed with the LZ-Y52 catalyst, but the roduction levels of the undesirable byproducts $CS_2$ and dimethyl sulfide, increased to an unacceptable level. The catalyst was found to contain coke and tars on removal from the reactor. The results of runs (41—44) of this example are reported in Table 4.

**Example 3**

Example 1 is repeated, except that the catalyst is a commercial alumina (Alcoa F-1) doped with 5% potassium hydroxide, based on the weight of the alumina, prepared by dissolving the KOH in water just sufficient to wet the catalyst, followed by oven drying. The operating conditions are comparable to those in Tables 2 and 3, and, in run 46, the level of single-pass conversion of methyl mercaptan to DMDS (30%) was equal to that observed during the runs of Example 1. The results of runs (45—48) of this example are reported in Table 5.

TABLE 1
(Example 1)

| Run No. | Catalyst bed temp. | CH$_3$SH mole velocity | H$_2$S mole velocity | Sulfur mole velocity | Inerts mole velocity | Bottoms mole velocity | Gms. DMDS produced | Gm CS$_2$ produced | Gm. DMS produced | Per cent conversion to DMDS |
|---|---|---|---|---|---|---|---|---|---|---|
| 1 | 150 | 936 | 423 | 228 | 0 | 0 | 8591 | 259 | 0 | 20 |
| 2 | 151 | 932 | 437 | 237 | 0 | 0 | 9165 | 247 | 0 | 22 |
| 3 | 151 | 942 | 608 | 229 | 0 | 0 | 8199 | 326 | 0 | 20 |
| 4 | 148 | 970 | 578 | 255 | 0 | 0 | 9583 | 304 | 0 | 22 |
| 5 | 152 | 1029 | 555 | 244 | 0 | 52 | 12779 | 345 | 0 | 25 |
| 6 | 150 | 1070 | 494 | 250 | 0 | 67 | 15321 | 553 | 0 | 28 |
| 7 | 153 | 1098 | 499 | 242 | 0 | 66 | 15572 | 645 | 0 | 28 |
| 8 | 154 | 992 | 537 | 135 | 0 | 0 | 8970 | 0 | 0 | 19 |
| 9 | 162 | 1010 | 508 | 162 | 0 | 0 | 10840 | 0 | 0 | 23 |
| 10 | 160 | 999 | 487 | 216 | 0 | 0 | 12240 | 0 | 0 | 26 |
| 11 | 156 | 1000 | 523 | 211 | 0 | 0 | 10403 | 0 | 0 | 22 |
| 12 | 154 | 996 | 513 | 181 | 0 | 40 | 12504 | 0 | 0 | 27 |
| 13 | 149 | 1009 | 531 | 146 | 0 | 68 | 13450 | 0 | 0 | 28 |
| 14 | 152 | 998 | 513 | 146 | 0 | 91 | 15230 | 0 | 0 | 32 |

EP 0 202 420 B1

TABLE 2
(Example 1)

| Run No. | Catalyst bed temp. | CH₃SH mole velocity | H₂S mole velocity | Sulfur mole velocity | Inerts mole velocity | Gms DMDS produced | Gm. CS₂ produced | Gm. DMS produced | Per cent conversion to DMDS |
|---|---|---|---|---|---|---|---|---|---|
| 15 | 203 | 200 | 400 | 200 | 50 | 2008 | 2700 | 170 | 21 |
| 16 | 169 | 200 | 400 | 200 | 50 | 2513 | 733 | 70 | 27 |
| 17 | 186 | 200 | 400 | 200 | 300 | 2500 | 1292 | 162 | 27 |
| 18 | 168 | 225 | 400 | 200 | 300 | 3351 | 582 | 50 | 31 |
| 19 | 160 | 203 | 400 | 200 | 300 | 2911 | 581 | 86 | 31 |
| 20 | 158 | 96 | 200 | 100 | 150 | 1019 | 510 | 68 | 22 |
| 21 | 151 | 88 | 800 | 100 | 0 | 14 | 1996 | 5 | 1 |
| 22 | 140 | 93 | 800 | 100 | 0 | 0 | 2065 | 0 | 0 |
| 23 | 166 | 222 | 400 | 200 | 300 | 449 | 4021 | 762 | 4 |

TABLE 3
(Example 1)

| Run No. | Catalyst bed temp. | CH₃SH mole velocity | H₂S mole velocity | Sulfur mole velocity | Inerts mole velocity | Gms. DMDS produced | Gm. CS₂ produced | Gm. DMS produced | Per cent conversion to DMDS |
|---|---|---|---|---|---|---|---|---|---|
| 24 | 167 | 208 | 400 | 245 | 400 | 384 | 542 | 46 | 4 |
| 25 | 168 | 200 | 400 | 243 | 400 | 876 | 543 | 10 | 9 |
| 26 | 166 | 206 | 400 | 215 | 400 | 1106 | 1213 | 2 | 11 |
| 27 | 165 | 204 | 400 | 220 | 400 | 1795 | 1587 | 0 | 19 |
| 28 | 165 | 202 | 400 | 179 | 400 | 2185 | 256 | 0 | 23 |
| 29 | 167 | 330 | 1335 | 362 | 400 | 1632 | 2945 | 0 | 11 |
| 30 | 167 | 308 | 1530 | 156 | 400 | 3232 | 1903 | 0 | 22 |
| 31 | 147 | 136 | 1000 | 62 | 300 | 544 | 462 | 0 | 8 |
| 32 | 136 | 140 | 1000 | 60 | 300 | 448 | 447 | 0 | 7 |
| 33 | 195 | 90 | 667 | 44 | 200 | 278 | 427 | 8 | 7 |
| 34 | 195 | 266 | 818 | 120 | 300 | 268 | 3618 | 0 | 2 |
| 35 | 136 | 178 | 545 | 66 | 200 | 904 | 752 | 0 | 11 |
| 36 | 195 | 267 | 818 | 107 | 300 | 1725 | 1052 | 0 | 14 |
| 37 | 135 | 273 | 818 | 109 | 300 | 1688 | 905 | 0 | 13 |
| 38 | 195 | 182 | 545 | 79 | 200 | 1229 | 956 | 0 | 14 |
| 39 | 135 | 86 | 667 | 41 | 200 | 277 | 238 | 0 | 7 |
| 40 | 195 | 139 | 1000 | 60 | 300 | 349 | 584 | 0 | 5 |

TABLE 4
(Example 2)

| Run No. | Catalyst bed temp. | CH₃SH mole velocity | H₂S mole velocity | Sulfur mole velocity | Inerts mole velocity | Gms. DMDS produced | Gm. CS₂ produced | Gm. DMS produced | Per cent conversion to DMDS |
|---|---|---|---|---|---|---|---|---|---|
| 41 | 264 | 100 | 800 | 100 | 0 | 12 | 0 | 220 | <1 |
| 42 | 264 | 100 | 800 | 420 | 0 | 152 | 2765 | 238 | 3 |
| 43 | 219 | 200 | 800 | 420 | 0 | 522 | 1198 | 93 | 6 |
| 44 | 207 | 300 | 800 | 420 | 0 | 3086 | 1881 | 344 | 22 |

TABLE 5
(Example 3)

| Run No. | Catalyst bed temp. | CH₃SH mole velocity | H₂S mole velocity | Sulfur mole velocity | Inerts mole velocity | Gms. DMDS produced | Gm. CS₂ produced | Gm. DMS produced | Per cent conversion to DMDS |
|---|---|---|---|---|---|---|---|---|---|
| 45 | 150 | 204 | 400 | 338 | 300 | 354 | 1703 | 91 | 4 |
| 46 | 190 | 204 | 400 | 400 | 300 | 2844 | 1819 | 623 | 30 |
| 47 | 191 | 210 | 400 | 400 | 300 | 1250 | 2866 | 92 | 13 |
| 48 | 187 | 184 | 400 | 400 | 300 | 1420 | 3236 | 99 | 17 |

**Claims**

1. A process for preparing di($C_1$—$C_{12}$)alkyl disulfides comprising continuously reacting a $C_1$—$C_{12}$ alkyl alcohol and hydrogen sulfide in a molar ratio of 1:2 to 1:20 in contact with a solid, particulate catalyst in a first reaction zone at a temperature ranging from 100 to 500°C, at a pressure ranging between atmospheric and 35 bar (500 psig) and a molar velocity of between 50 and 500 gram-moles of alcohol per kilogram of catalyst per 24 hours, continuously passing the crude mercaptan product of the reaction and molten sulfur at a molar ratio of from 1:0.05 to 1:2 in contact with a solid, particulate catalyst in a second reaction zone at a temperature ranging from 125 to 400°C, and pressure ranging from atmospheric to 42.5 bar (600 psig) and recovering the di($C_1$—$C_{12}$)alkyl disulfide, wherein the catalyst in the second reaction zone is a Type X, Y or L zeolite.

2. The process of claim 1 wherein the reaction in said first reaction zone is at a temperature in the range of from 250 to 400°C, a pressure of from atmospheric to 35 bar (500 psig) and a molar velocity of between 50 and 500 gram-moles of alcohol per kilogram of catalyst per 24 hours.

3. The process of claims 1 or 2 wherein the reaction in the second reaction zone is at a temperature in the range of 125 to 225°C, a pressure of from atmospheric to 42 bar (600 psig), and a molar velocity of 100 to 2000 gram-moles of alkyl mercaptan per kilogram of catalyst per 24 hours.

4. The process of claims 1, 2 or 3 wherein the catalyst in each reaction zone is an aluminum-containing catalyst.

5. The process of claim 1 wherein said catalyst is a Type X or Y zeolite containing from 3 to 13% by weight of alkali metal, expressed as $Me_2O$.

6. The process of claim 1 wherein said catalyst is a Type Y zeolite containing from 5 to 13% by weight of sodium, expressed as $Na_2O$.

7. The process of claim 1 comprising continuously reacting a $C_1$—$C_4$ alkyl alcohol and hydrogen sulfide in a molar ratio of 1:6 to 1:10 in contact with an aluminum-containing, solid, particulate catalyst in a first reaction zone at a temperature ranging from 250 to 400°C at a pressure of between 6.9 and 27.5 bar (100 and 400 psig) and a molar velocity of between 100 and 150 gram-moles of alcohol per kilogram of catalyst per 24 hours, continuously passing the crude mercaptan product of the reaction and molten sulfur at a molar ratio of from 1:0.05 to 1:1 in contact with a Type X or Y zeolite catalyst in a second reaction zone at a temperature ranging from 125 to 225°C, at a pressure within the range of 3.4 to 25.8 bar (50 to 375 psig) and at a molar velocity in the range of 750 to 1250 gram-moles of alkyl mercaptan per kilogram of catalyst per 24 hours, and recovering a di($C_1$—$C_4$)alkyl disulfide.

8. The process of claim 7 wherein the crude di($C_1$—$C_4$)alkyl disulfide product of the second reaction zone is distilled to remove one or more of the by-products di($C_1$—$C_4$)alkyl sulfide, carbon disulfide, hydrogen sulfide and di($C_1$—$C_4$)alkyl polysulfides, and one or more of these by-products is returned to the first or second reaction zones.


**Patentansprüche**

1. Verfahren zur Herstellung von Di($C_1$—$C_{12}$)alkyldisulfiden, dadurch gekennzeichnet, daß man kontinuierlich einen $C_1$—$C_{12}$ Alkylalkohol und Schwefelwasserstoff in einem Molverhältnis von 1:2 bis 1:20 in Kontakt mit einem festen, teilchenförmigen Katalysator in einer ersten Reaktionszone bei einer Temperatur im Bereich von 100 bis 500°C bei einem Druck im Bereich zwischen Atmosphärendruck und 35 bar (500 psig) und einer Molgeschwindigkeit von zwischen 50 und 500 gramm-mol Alkohol pro Kilogramm Katalysator pro 24 Stunden umsetz, kontinuierlich das rohe Mercaptanprodukt der Reaktion und geschmolzenen Schwefel in einem Molverhältnis von 1:0,05 bis 1:2 in Kontakt mit einem festen, teilchenförmigen Katalysator in eine zweite Reaktionszone bei einer Temperatur im Bereich von 125 bis 400°C und einem Druck im Bereich von Atmosphärendruck bis 42,5 bar (600 psig) leitet und das Di($C_1$—$C_{12}$)alkyldisulfid gewinnt, wobei der Katalysator in der zweiten Reaktionszone ein X-, Y- oder L-Typ-Zeolith ist.

2. Verfahren nach Anspruch 1, dadurch gekennzeichnet, daß die Reaktion in der ersten Reaktionszone bei einer Temperatur im Bereich von 250 bis 400°C, einem Druck von Atmosphärendruck bis 35 bar (500 psig) und einer Molgeschwindigkeit zwischen 50 und 500 gramm-mol Alkohol pro Kilogramm Katalysator pro 24 Stunden stattfindet.

3. Verfahren nach Anspruch 1 oder 2, dadurch gekennzeichnet, daß die Reaktion in der zweiten Reaktionszone bei einer Temperatur im Bereich von 125 bis 225°C, einem Druck von Atmosphärendruck bis 42 bar (600 psig) und einer Molgeschwindigkeit von 100 bis 2000 gramm-mol Alkylmercaptan pro Kilogramm Katalysator pro 24 Stunden stattfindet.

4. Verfahren nach Anspruch 1, 2 oder 3, dadurch gekennzeichnet, daß der Katalysator in jeder Reaktionszone ein aluminiumhaltiger Katalysator ist.

5. Verfahren nach Anspruch 1, dadurch gekennzeichnet, daß der Katalysator ein X- oder Y-Typ-Zeolith, der von 3 bis 13 Gewichts-% Alkalimetall, ausgedrückt als $Me_2O$, enthält, ist.

6. Verfahren nach Anspruch 1, dadurch gekennzeichnet, daß der Katalysator ein Y-Typ-Zeolith, der von 5 bis 13 Gewichts-% Natrium, ausgedrückt als $Na_2O$, enthält, ist.

7. Verfahren nach Anspruch 2, dadurch gekennzeichnet, daß man kontinuierlich einen $C_1$—$C_4$-

Alkylalkohol und Schwefelwasserstoff in einem Molverhältnis von 1:6 bis 1:10 in Kontakt mit einem aluminiumhaltigen, festen, teilchenförmigen Katalysator in einer ersten Reaktionszone bei einer Temperatur im Bereich von 250 bis 400°C bei einem Druck von zwischen 6,9 und 27,5 bar (100 und 400 psig) und einer Molgeschwindigkeit von zwischen 100 und 150 gramm-mol Alkohol pro Kilogramm Katalysator pro 24 Stunden umsetzt, kontinuierlich das rohe Mercaptanprodukt der Reaktion und geschmolzenen Schwefel in einem Molverhältnis von 1:0,05 bis 1:1 in Kontakt mit einem X- oder Y-Typ-Zeolithkatalysator in eine zweite Reaktionszone bei einer Temperatur im Bereich von 125 bis 225°C, bei einem Druck im Bereich von 3,4 bis 25,8 bar (50 bis 375 psig) und einer Molgeschwindigkeit im Bereich von 750 bsi 1250 gramm-mol Alkylmercaptan pro Kilogramm Katalysator pro 24 Stunden leitet und daß man ein Di($C_1$—$C_4$)alkyldisulfid gewinnt.

8. Verfahren nach Anspruch 7, dadurch gekennzeichnet, daß das rohe Di($C_1$—$C_4$)alkyldisulfidprodukt der zweiten Reaktionszone destilliert wird, um eines oder mehrere der Nebenprodukte, Di($C_1$—$C_4$)alkyldisulfid, Kohlenstoffdisulfid, Schwerfelwasserstoff und Di($C_1$—$C_4$)alkylpolysulfide zu entfernen und, daß eines oder mehrere dieser Nebenprodukte in die erste oder zweite Reaktionszone zurückgeführt werden.

## Revendications

1. Procédé de préparation de disulfures de di(alkyle en $C_1$ à $C_{12}$), qui consiste à faire réagir en continu un alcool alkylique en $C_1$ à $C_{12}$ et du sulfur d'hydrogène dans un rapport molaire de 1:2 à 1:20 au contact d'un catalyseur solide en particules dans une première zone de réaction à une température comprise dans la plage de 100 à 500°C, à une pression allant de la pression atmosphérique à 35 bars (500 lb/in$^2$ au manomètre) et à une vitesse molaire comprise entre 50 et 500 molécules-grammes d'alcool par kilogramme de catalyseur par 24 heures, à faire passer en continu le mercaptan obtenu comme produit réactionnel brut et du soufre fondu dans un rapport molaire de 1:0,05 à 1:2 au contact d'un catalyseur solide en particules dans une seconde zone de réaction à une température allant de 125 à 400°C et à une pression allant de la pression atmosphérique à 42,5 bars (600 lb/in$^2$ au manomètre), et à recueillir le disulfure de di(alkyl en $C_1$ à $C_{12}$), le catalyseur dans la seconde zone de réaction étant une zéolite de type X, Y ou L.

2. Procédé suivant la revendication 1, dans lequel la réaction dans ladite première zone de réaction s'effectue à une température comprise dans la plage de 250 à 400°C, à une pression allant de la pression atmosphérique à 35 bars (500 lb/in$^2$ au manomètre) et à une vitesse molaire comprise entre 50 et 500 molécules-grammes d'alcool par kilogramme de catalyseur par 24 heures.

3. Procédé suivant les revendications 1 ou 2, dans lequel la réaction dans la seconde zone de réaction est conduite à une température comprise dans la plage de 125 à 225°C, à une pression allant de la pression atmosphérique à 42 bars (600 lb/in$^2$ au manomètre) et à une vitesse molaire de 100 à 2000 molécules-grammes d'alkylmercaptan par kilogramme de catalyseur par 24 heures.

4. Procédé suivant les revendications 1, 2 ou 3, dans lequel le catalyseur dans chaque zone de réaction est un catalyseur contenant de l'aluminum.

5. Procédé suivant la revendication 1, dans lequel le catalyseur est une zéolite de type X ou Y contenant 3 à 13% en poids de métal alcalin, exprimé en $Me_2O$.

6. Procédé suivant la revendication 1, dans lequel le catalyseur est une zéolite du type Y contenant 5 à 13% en poids de sodium, exprimé en $Na_2O$.

7. Procédé suivant la revendication 1, qui consiste à faire réagir en continu un alcool alkylique en $C_1$ à $C_4$ et du sulfure d'hydrogène dans un rapport molaire de 1:6 à 1:10 au contact d'un catalyseur solide en particules contenant de l'aluminium dans une première zone de réaction à une température allant de 250 à 400°C, sous une pression comprise entre 6,9 et 27,5 bars (100 à 400 lb/in$^2$ au manomètre) et à une vitesse molaire comprise entre 100 et 150 molécules-grammes d'alcool par kilogramme de catalyseur par 24 heures, à faire passer en continu le mercaptan obtenu comme produit réactionnel brut et du soufre fondu dans un rapport molaire de 1:0,05 à 1:1 au contact d'un catalyseur zéolitique de type X ou Y dans une seconde zone de réaction à une température allant de 125 à 225°C, à une pression comprise dans la plage de 3,4 à 25,8 bars (50 à 375 lb/in$^2$ au manomètre) et à une vitesse molaire comprise dans la plage de 750 à 1250 molécules-grammes d'alkylmercaptan par kilogramme de catalyseur par 24 heures, et à recueillir un disulfure de di(alkyl en $C_1$ à $C_4$).

8. Procédé suivant la revendication 7, dans lequel le disulfure de di(alkyle en $C_1$ à $C_4$) brut obtenu comme produit dans la seconde zone de réaction est distillé pour en éliminer un ou plusieurs des sous-produits, à savoir sulfur de di(alkyle en $C_1$ à $C_4$), sulfure de carbone, sulfur d'hydrogène et polysulfures de di(alkyle en $C_1$ à $C_4$), et un ou plusieurs de ces sous-produits sont recyclés à la première ou à la seconde zone de réaction.